(19) Europäisches Patentamt
European Patent Office
Office européen des brevets

(11) Publication number: **0 145 179**
**B1**

# (12) EUROPEAN PATENT SPECIFICATION

(45) Date of publication of patent specification: **23.11.88**

(51) Int. Cl.$^4$: **C 07 D 213/64,** A 01 N 53/00

(21) Application number: **84307125.9**

(22) Date of filing: **17.10.84**

## (54) Halo((phenoxypyridyl)methyl)esters, process for their preparation and their use as insecticides.

(30) Priority: **18.10.83 GB 8327793**

(43) Date of publication of application:
**19.06.85 Bulletin 85/25**

(45) Publication of the grant of the patent:
**23.11.88 Bulletin 88/47**

(84) Designated Contracting States:
**AT BE CH DE FR IT LI LU NL SE**

(56) References cited:
**EP-A-0 050 846**
**EP-A-0 076 961**
**FR-A-2 383 927**

(73) Proprietor: **IMPERIAL CHEMICAL INDUSTRIES PLC**
**Imperial Chemical House Millbank**
**London SW1P 3JF (GB)**

(72) Inventor: **Bushell, Michael John**
**21 Suffolk Close Woosehill**
**Wokingham Berkshire, RG11 9AU (GB)**
Inventor: **Fraser, Alastair Mclaren**
**51 Pembrook Hanworth**
**Bracknell Berkshire (GB)**

(74) Representative: **Bishop, Nigel Douglas et al**
**Imperial Chemical Industries PLC Legal Department: Patents PO Box 6 Bessemer Road**
**Welwyn Garden City Herts, AL7 1HD (GB)**

**EP 0 145 179 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel pyrethroid compounds characterised by good residual insecticidal activity in some cases combined with an unexpected rapid "knock-down" effect on flying insect pests e.g. houseflies and mosquitos.

UK Patent No. 1572149 discloses phenoxypyridylmethyl esters of dihalovinylcyclopropane carboxylic acids, and in particular discloses compounds of formula:

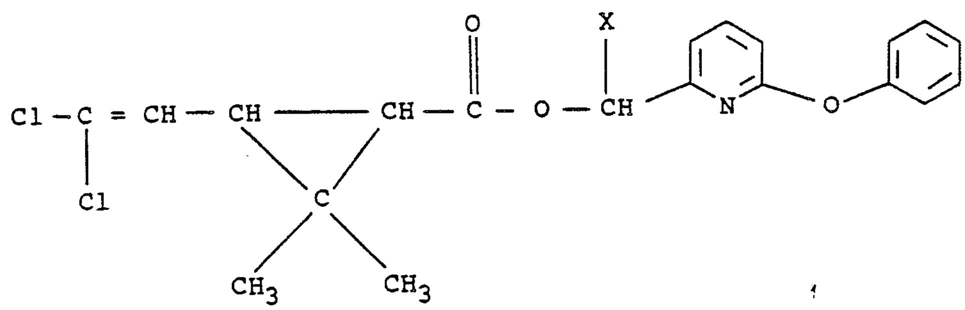

wherein X is hydrogen, cyano or ethynyl.

We have now discovered that compounds wherein one of the chloro substituents is replaced by trifluoromethyl have surprising better activity than these known compounds.

Accordingly, the present invention provides compounds (including single isomers thereof and mixtures of these isomers, including racemates) of formula:

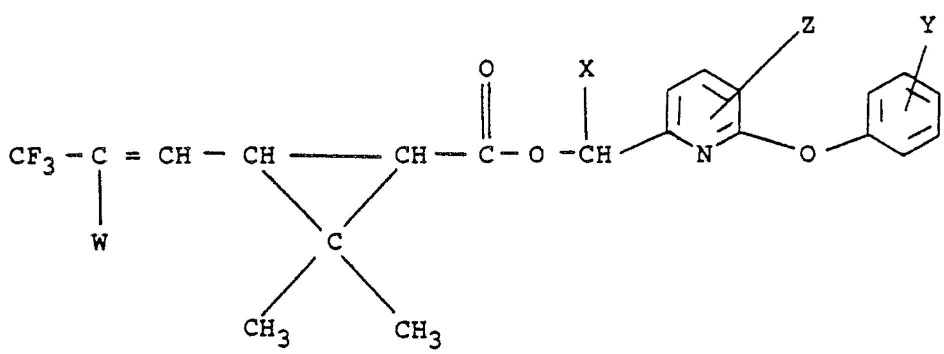

(I)

wherein W is chloro, bromo or fluoro, preferably chloro, X is hydrogen, ethynyl or cyano, Y represents hydrogen, chloro, bromo or fluoro, and Z represents hydrogen, chloro or fluoro.

Preferably the compounds of formula I are those wherein X is hydrogen or cyano, Y is hydrogen, fluoro or chloro and Z is hydrogen. More preferably X is hydrogen or cyano and Y and Z are both hydrogen. When Y is not hydrogen it is preferably in the 4-position.

Examples of compounds according to the invention include those according to formula I as set out in Table I below wherein the values of W, X, Y and Z are given for each compound.

TABLE I

| COMPOUND NO | W | X | Y | Z |
|---|---|---|---|---|
| 1 | Cl | H | H | H |
| 2 | Cl | CN | H | H |
| 3 | Cl | H | 4-F | H |
| 4 | F | H | H | H |
| 5 | Cl | H | 4-Cl | H |
| 6 | Br | H | H | H |
| 7 | Br | CN | H | H |
| 8 | F | CN | H | H |
| 9 | Cl | CN | 4-Cl | H |
| 10 | Br | H | 4-F | H |
| 11 | Cl | CN | 4-F | H |
| 12 | Cl | CN | H | 5-F |
| 13 | Cl | C≡CH | H | H |

It will be appreciated that the compounds of formula I may exist in different isomeric forms. Isomerism arises from the possibility of *E* and *Z* substitution patterns in the 3-alkenyl substituent on the cyclopropane ring, thus

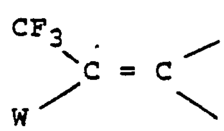

and

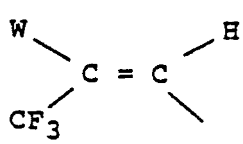

and from the presence of two chiral centres on the cyclopropane ring (at the 1- and 3-positions), plus the possibility in compounds where X is not hydrogen of a further chiral centre at the α-carbon in the alcohol moiety. Thus each of these chiral centres may be in the (R) or (S) configuration. In the case of the cyclopropane ring this imposes either a *cis* or *trans* configuration on the disposition of the hydrogen atoms at the 1- and 3-positions. For convenience the isomerism or isomer content of products according to the invention is indicated herein by indication of the absolute stereochemistry of the 1-position in combination with with the designation *cis* or *trans* to identify the particular isomer involved, for example (1R,*cis*) for a single isomer, (1RS,*cis*) for a racemic mixture of *cis* isomers, etc. Because some compounds of the invention may comprise a mixture of isomers, individual embodiments as described hereinafter are designated as "Products", as follows:

Product I: 6-phenoxypyrid-2-ylmethyl (1RS,*cis*)-3-(*Z*-2-chloro-3,3,3-trifluoroprop-1-en-1-yl)-2,2-dimethylcyclopropane carboxylate.

Product II: (RS)-1-cyano-1-(6-phenoxypyrid-2-yl)methyl (1RS,*trans*)-3-(*Z*-2-chloro-3,3,3-trifluoroprop-1-en-1-yl)-2,2-dimethylcyclopropane carboxylate.

Product III: Mixture of Product II and Product IV (1:1).

Product IV: (RS)-1-cyano-1-(6-phenoxypyrid-2-yl)methyl (1RS,*cis*)-3-(*Z*-2-chloro-3,3,3-trifluoroprop-1-en-1-yl)-2,2-dimethylcyclopropane carboxylate.

Product V: 6-phenoxypyrid-2-ylmethyl (1RS,*trans*)-3-(*Z*-2-chloro-3,3,3-trifluoroprop-1-en-1-yl)-2,2-dimethylcyclopropane carboxylate.

Product VI: 6-(4-fluorophenoxy)pyrid-2-ylmethyl (1RS,*cis*)-3-(Z-2-chloro-3,3,3-trifluoroprop-1-en-1-yl)-2,2-dimethylcyclopropane carboxylate.

Product VII: Mixture of 6-phenoxypyrid-2-ylmethyl (1RS,*cis*)-3-(*E*-2,3,3,3-tetrafluoroprop-1-en-1-yl)-2,2-dimethylcyclopropane carboxylate, 6-phenoxypyrid-2-ylmethyl (1RS,*trans*)-3-(*E*-2,3,3,3-tetrafluoroprop-1-en-1-yl)-2,2-dimethylcyclopropane carboxylate, Product XI, and 6-phenoxypyrid-2-ylmethyl (1RS,*trans*)-3-(*Z*-2,3,3,3-tetrafluoroprop-1-en-1-yl)-2,2-dimethylcyclopropane carboxylate (3:6:22:61).

Product VIII: (6-(4-chlorophenoxy)pyrid-2-ylmethyl (1RS,*cis)*-3-(*Z*-2-chloro-3,3,3-trifluoroprop-1-en-1-yl)-2,2-dimethylcyclopropane carboxylate.

Product IX: 6-phenoxypyrid-2-ylmethyl (1RS,*cis*)-3-(*Z*-2-bromo-3,3,3-trifluoroprop-1-en-1-yl)-2,2-dimethylcyclopropane carboxylate.

Product X: (RS)-1-cyano-1-(6-phenoxypyrid-2-yl)methyl (1RS,*cis*)-3-(*Z*-2-bromo-3,3,3-trifluoroprop-1-en-1-yl)-2,2-dimethylcyclopropane carboxylate.

Product XI: 6-phenoxypyrid-2-ylmethyl (1RS,*cis*)-3-(*Z*-2,3,3,3-tetrafluoroprop-1-en-1-yl)-2,2-dimethylcyclopropane carboxylate.

Product XII: (RS)-1-cyano-1-(6-phenoxypyrid-2-yl)methyl (1RS,*cis*)-3-(*Z*-2,3,3,3-tetrafluoroprop-1-en-1-yl)-2,2-dimethylcyclopropane carboxylate.

Product XIII: (RS)-1-cyano-1-[6-(4-chlorophenoxy)pyrid-2-yl]methyl (1RS,*cis*)-3-(*Z*-2-chloro-3,3,3-trifluoroprop-1-en-1-yl)-2,2-dimethylcyclopropane carboxylate.

Product XIV: 6-(4-fluorophenoxy)pyrid-2-ylmethyl (1RS,*cis*)-3-(*Z*-2-bromo-3,3,3-trifluoroprop-1-en-1-yl)-2,2-dimethylcyclopropane carboxylate.

Product XV: (RS)-1-cyano-1-[6-(4-fluorophenoxy)pyrid-2-yl]methyl (1RS,*cis*)-3-(*Z*-2-chloro-3,3,3-trifluoroprop-1-en-1-yl)-2,2-dimethylcyclopropane carboxylate.

Product XVI: A mixture of (S)-1-cyano-1-(6-phenoxypyrid-2-yl)methyl (1R,*cis*)-3-(*Z*-2-chloro-3,3,3-trifluoroprop-1-en-1-yl)-2,2-dimethylcyclopropane carboxylate and (R)-1-cyano-1-(6-phenoxypyrid-2-yl)methyl (1R,*cis*)-3-(*Z*-2-chloro-3,3,3-trifluoroprop-1-en-1-yl)-2,2-dimethylcyclopropane carboxylate (85:12).

Product XVII: The mixture of isomers named in Product XVI in the ratio 3:47.

Product XVIII: A mixture of (S)-1-cyano-1-(6-phenoxypyrid-2-yl)methyl (1R,*cis*)-3-(*Z*-2-chloro-3,3,3-trifluoroprop-1-en-1-yl)-2,2-dimethylcyclopropane carboxylate, (R)-1-cyano-1-(6-phenoxypyrid-2-yl)methyl (1S,*cis*)-3-(*Z*-2-chloro-3,3,3-trifluoroprop-1-en-1-yl)-2,2-dimethylcyclopropane carboxylate, (R)-1-cyano-1-(6-phenoxypyrid-2-yl)methyl (1R,*cis*)-3-(*Z*-2-chloro-3,3,3-trifluoroprop-1-en-1-yl)-2,2-dimethylcyclopropane carboxylate, and (S)-1-cyano-1-(6-phenoxypyrid-2-yl)methyl (1S,*cis*)-3-(*Z*-2-chloro-3,3,3-trifluoroprop-1-en-1-yl)-2,2-dimethylcyclopropane carboxylate (2:2:23:23).

Product XIX: The mixture of isomers named in Product XVIII in the ratio 93:93:7:7.

Product XX: (RS)-1-cyano-1-(6-phenoxypyrid-2-yl)methyl (1R,*cis*)-3-(*Z*-2-chloro-3,3,3-trifluoroprop-1-en-1-yl)-2,2-dimethylcyclopropane carboxylate.

The compounds of the invention according to Formula I are esters and may be prepared by conventional esterification processes, of which the following are examples.

(a) An acid of formula:

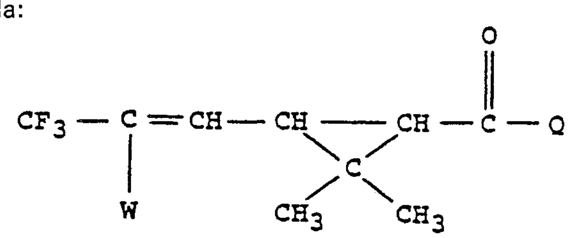

where Q represents the hydroxy group and W has any of the meanings given hereinabove, may be reacted directly with an alcohol of formula:—

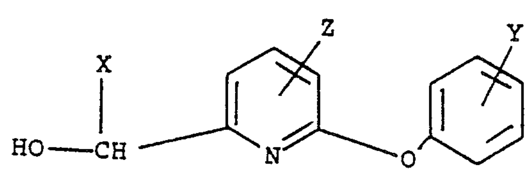

(III)

wherein X, Y and Z have any of the meanings given herein above, the reaction preferably taking place in the presence of an acid catalyst, for example, dry hydrogen chloride, or a dehydrating agent, for example, a carbodiimide such as dicyclohexylcarbodiimide.

(b) An acid halide of formula II where Q represents a halogen atom, preferably a chlorine atom, and W has any of the meanings given hereinabove, may be reacted with an alcohol of formula III, the reaction preferably taking place in the presence or a base, for example, pyridine, trialkylamine alkali metal hydroxide or carbonate, or alkali metal alkoxide.

(c) An acid of formula II where Q represents the hydroxy group and W has any of the meanings given hereinabove, or preferably, an alkali metal salt thereof, may be reacted with halide of formula:—

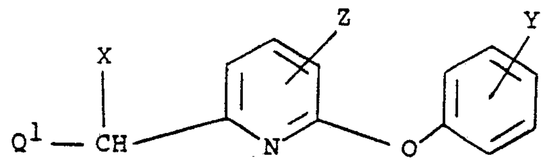

(IV)

where $Q^1$ represents a halogen atom, preferably the bromine or chlorine atom, and X, Y and Z have any of the meanings given hereinabove or with the quaternary ammonium salts derived from such halides with tertiary amines, for example pyridine, or trialkylamines such as triethylamine.

(d) A lower alkyl ester of formula II where Q represents a lower alkoxy group containing up to six carbon atoms, preferably the methoxy or ethoxy group, and W has any of the meanings given hereinabove, is heated with an alcohol of formula III to effect a transesterification reaction. Preferably the process is performed in the presence of a suitable catalyst, for example, an alkali metal alkoxide, such as sodium methoxide, or an alkylated titanium derivative, such as tetramethyl titanate.

All of these conventional processes for the preparation of esters may be carried out using solvents and diluents for the various reactants where appropriate, and may be accelerated or lead to higher yields of product when performed at elevated temperatures or in the presence of appropriate catalysts, for example phase-transfer catalysts.

The preparation of individual isomers may be carried out in the same manner but commencing from the corresponding individual isomers of compounds of formula II. These may be obtained by conventional isomer separation techniques from mixtures of isomers. Thus *cis* and *trans* isomers may be separated by fractional crystallisation of the carboxylic acids or salts thereof, whilst the various optically active species may be obtained by fractional crystallisation of salts of the acids with optically active amines, followed by regeneration of the optically pure acid. The optically pure isomeric form of the acid (or its equivalent acid chloride or ester) may then be reacted with the appropriate alcohol to produce a compound of formula I in the form of an individually pure isomer thereof.

The preparation of the compounds of formula II wherein Q is hydroxy, alkoxy or halo, and W is as defined hereinabove, useful as intermediates in the preparation of the compounds of the invention, is fully described in British Patent Specification 2,000,764 and in US patent No 4,183,948.

The compounds of formulae (III) are disclosed in US Patent No 4,256,890. The compounds of formula (IV) may be prepared by reducing the compounds of formula:

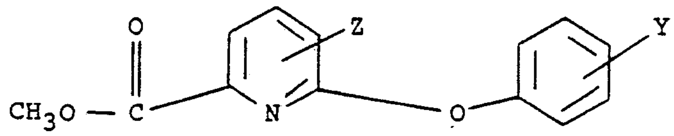

(known from US Patent 4,256,890) with e.g. hydrogen over a copper or palladium catalyst to yield the corresponding compounds of formula:

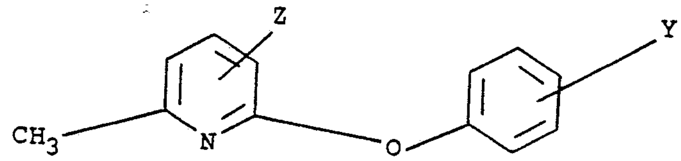

which are then halogenated with e.g. N-halosuccinimide.

The compounds of formula I may be used to combat and control infestations of insect pests and also other invertebrate pests, in particular, acarine pests. The insect and acarine pests which may be combatted and controlled by the use of the invention compounds include those pests associated with agriculture (which term includes the growing of crops for food and fibre products, horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise a insecticide synergist, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the compositions may be in the form of liquid preparations to be used as dips or sprays, which are generally aqueous dispersions or emulsions of the active ingredient in the presence of one or more known wetting agents, dispersing agents or emulsifying agents (surface active agents).

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters or sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl- and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents. Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydro furfuryl alcohol (THFA).

The compositions to be used as sprays may also be in the form of aerosols wherein the formulation is held in a container under pressure in the presence of a propellant such as fluorotrichloromethane or dichlorodifluoromethane.

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogenous for a sufficient time to enable them to be applied by conventional spray equipment. The concentrates may contain 10—85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compositions of the invention are very toxic to wide varieties of insect and other invertebrate pests, including, for example, the following:—

*Aphis fabae* (aphids)
*Megoura viceae* (aphids)
*Musca domestica* (houseflies)
*Blattella germanica* (cockroaches)
*Aedes aegypti* (mosquitos)
*Plutella xylostella* (diamond back moth, larvae)
*Tetranychus cinnabarinus* (carmine spider mite)
*Tetranychus urticae* (red spider mites)
*Panonychus ulmi* (citrus rust mite)
*Diabrotica* spp. (rootworms)
*Heliothis virescens* (tobacco budworms)
*Chilo partellus* (stem borers)

The compounds of formula I and compositions comprising them have shown themselves to be particularly useful in controlling foliar feeding pests of plants, and public health pests such as flies and mosquitos. The compounds may also be used to combat pests which inhabit the soil, for example *Diabrotica* spp. They may also be useful in combatting insect and acarine pests which infest domestic animals, such as *Lucilia sericata,* and ixodid ticks such as *Boophilus* spp., *Ixodes* spp., *Amblyomma* spp.,

*Rhipicephalus* spp., and *Dermocentor* spp. They are expected to be effective in combatting both susceptible and resistant strains of these pests in their adult, larval and intermediate stages of growth, and may be applied to the infested host animal by topical, oral or parenteral administration.

In particular, the invention compounds have useful knock-down properties which make them of value as ingredient in aerosol and like sprays designed for use against flying insect pests such as houseflies, mosquitos and the like. In such sprays they may be used on their own or in conjunction with other insecticidal component. However, unlike other knock-down agents such as tetramethrin and allethrin the invention compounds combine good knock-down properties with a lethal effect at the same concentration.

In the following examples, which further illustrate the invention, the novel compounds produced were examined by infra-red and nuclear-magnetic spectroscopy, and gave spectra consistent with the indicated structures of the compounds.

Example 1

This Example illustrates the preparation of 6-phenoxypyrid-2-ylmethyl (1RS,*cis*)-3-(*Z*-2-chloro-3,3,3-trifluoroprop-1-en-1-yl)-2,2-dimethylcyclopropane carboxylate.

A solution of 6-phenoxypyrid-2-ylmethanol (0.71 g) in toluene (10 ml) was added at the ambient temperature (ca. 20°C) to a stirred mixture of (1RS,*cis*)-1-chlorocarbonyl-3-(Z-2-chloro-3,3,3-trifluoroprop-1-en-1-yl)-2,2-dimethylcyclopropane (0.97 g), pyridine (0.3 g) and toluene (10 ml) and the mixture stirred for a further 3 hours, after which it was allowed to stand for 3 days. The mixture was then diluted with water and extracted with diethyl ether. The ethereal extracts were dried over anhydrous magnesium sulphate and concentrated by evaporation of the solvent under reduced pressure and the residual oil purified by column chromatography using a silica gel column and a 3% v/v solution of methanol in chloroform as eluent. There was thus obtained 6-phenoxypyrid-2-ylmethyl (1RS,*cis*)-3-(*Z*-2-chloro-3,3,3-trifluoroprop-1-en-1-yl)-2,2-dimethylcyclopropane (1.18 g) as a colourless oil.

NMR ($CDCl_3$) δ: 1.3 (m, 6H); 2.0—2.4 (m, 2H); 5.05 (s, 2H); 6.6—7.7 (m, 9H)
Infra red (liquid film): 1734, 1600, 1583, 1496, 1455, 1300, 1280, 1205, 1144 $cm^{-1}$

Example 2

This Example illustrates the preparation of (RS)-1-cyano(6-phenoxypyrid-2-yl)methyl (1RS,*cis*)-3-(*Z*-2-chloro-3,3,3-trifluoroprop-1-en-1-yl)-2,2-dimethylcyclopropane carboxylate (Product XV)

(a) Preparation of (RS)-1-cyano-1-[6-(4-fluorophenoxy) pyrid-2-yl]methanol.

A solution of potassium cyanide (0.28 g) in water (5 $cm^3$) was added to a vigorously stirred mixture of 2-formyl-6-(4-fluorophenoxy)pyridine (1.25 g) and glacial acetic acid (10 $cm^3$) at the ambient temperature and the mixture stirred for 5 hours and then kept for 18 hours without stirring. Care was taken to remove any hydrogen cyanide vapours vented from the mixture by the use of a trap containing sodium hypochlorite. The mixture was extracted with chloroform (3 × 25 $cm^3$) and the combined extracts dried over anhydrous magnesium sulphate and concentrated by removal of chloroform under reduced pressure. The oily residue was purified by column chromatography to remove unreacted starting material and give (RS)-1-cyano-1-[6-(4-fluorophenoxy)pyrid-2-yl-methanol (1.15 g).

NMR ($CDCl_3$) δ: 2.2—3.2 (broad, 1H); 5.41 (s, 1H); 6.85—7.95 (m, 7H)
Infra red (liquid film): 3600—3100, 1600, 1590, 1580, 1490, 1010, 830, 805 $cm^{-1}$

(b) Preparation of Product XV

A mixture of (RS)-1-cyano-1-[6-(4-fluorophenoxy)pyrid-2-yl] methanol (200 mg), (1RS,*cis*)-3-(*Z*-2-chloro-3,3,3-trifluoroprop-1-en-1-yl)-2,2-dimethylcyclopropane carboxylic acid (250 mg), 4-dimethylaminopyridine (5 mg), dicyclohexylcarbodiimide (210 mg) and dry dichloromethane (5 $cm^3$) was stirred at the ambient temperature for 2 hours. After removal of the solid precipitate by filtration, this filtrate was concentrated by evaporation of the solvent and the residual oil purified by column chromatography to yield (RS)-1-cyano-1-[6-(4-fluorophenoxy)pyrid-2-yl]methyl (1RS,cis)-3-(*Z*-2-chloro-3,3,3-trifluoroprop-1-en-1-yl)-2,2-dimethylcyclopropane carboxylate (260 mg) as a viscous oil.

Example 3

By the procedure of either Example 1 or Example 2(b) the other Products were obtained by reaction of the appropriate alcohols with either the acid or acid chloride. The following table sets out the n.m.r. and infra red data for the various Products obtained.

| PRODUCT | METHOD OF EXAMPLE | NMR ($CDCl_3$) | INFRA RED ($cm^{-1}$) (LIQUID FILM) |
|---|---|---|---|
| II | 1 | 1.2-1.4(m,6H)<br>1.85(d,1H)<br>2.45(m,1H)<br>6.1 (d,1H)<br>6.30 (s,1H)<br>6.85-7.8(m,8H) | 1750, 1600,<br>1580, 1498,<br>1286, 1228,<br>1200, 1150 |
| III | 1 | 1.15-1.4(m,6H)<br>1.75-2.5(m,2H)<br>6.1(d,0.5H)<br>6.2-6.25(m,1H)<br>6.7-7.8(m,8.5H) | 1745, 1596,<br>1578, 1494,<br>1448, 1276,<br>1200, 1130 |
| IV | 1 | 1.2-1.4(m,6H)<br>2.0-2.4(m,2H)<br>6.3(d,1H)<br>6.75-7.9(m,9H) | 1750, 1600,<br>1580, 1496,<br>1452, 1280,<br>1200, 1135,<br>1080 |
| V | 1 | 1.25(s,3H)<br>1.34(s,3H)<br>1.86(d,1H)<br>2.4(m,1H)<br>5.16(s,2H)<br>6.2(d,1H)<br>6.7-7.8(m,8H) | 1736, 1600,<br>1582, 1496,<br>1454, 1286,<br>1226, 1170 |

| PRODUCT | METHOD OF EXAMPLE | NMR ($CDCl_3$) | INFRA RED ($cm^{-1}$) (LIQUID FILM) |
|---|---|---|---|
| VI | 1 | 1.28(d,6H)<br>2.1-2.25(m,2H)<br>5.09(s,2H)<br>6.06-7.78(m,7H) | 3000-3850,<br>1735, 1600,<br>1580, 840 |
| VII | 1 | 1.22(s), 1.28(s), 1.31(s) } 6H<br>1.7-2.8(m,2H)<br>5.13(q,2H)<br>5.5(d,1H)<br>6.6-7.8(m,7H) | 3100-2900,<br>1740, 1600,<br>1580, 1500,<br>1455, 1355,<br>870, 850,<br>735, 700 |
| VIII | 1 | 1.28(d,6H)<br>2.02-2.3(q,2H)<br>5.08(s,2H)<br>6.7-7.8(m,8H) | 1735, 1600,<br>1595, 1580,<br>1490, 835 |
| IX | 1 | 1.29(d,6H)<br>2.05-2.3(m,2H)<br>5.11(s,2H)<br>6.7(d,1H)<br>6.95-7.75(m,8H) | 2930, 2915,<br>1735, 1595,<br>1580, 1490,<br>710, 690 |
| X | 1 | 1.27(d,6H)<br>1.8-2.3(m,2H)<br>6.28(d,1H)<br>6.9(d,1H)<br>7.0-7.8(m,8H) | 3070-2950,<br>1740, 1595,<br>1580, 1445,<br>760, 695 |

| PRODUCT | METHOD OF EXAMPLE | NMR ($CDCl_3$) | INFRA RED ($cm^{-1}$) (LIQUID FILM) |
|---|---|---|---|
| XI | 1 | 1.27(s,6H)<br>1.94-2.29(m,2H)<br>5.11(s,2H)<br>5.8, 5.9, 6.2, 6.3 (q,1H)<br>6.63-7.75(m,8H) | 2950, 1730, 1600, 1580, 1490, 1450, 850, 780, 700 |
| XII | 1 | 1.30(d,6H)<br>1.9-2.25(m,2H)<br>5.8, 6.1 (d,1H)<br>6.28(d,1H)<br>6.9-7.5(m,7H)<br>7.65-7.85(t,1H) | 3080, 2950, 1740, 1600, 1580, 1490, 850, 770, 700 |
| XIII | 1 | 1.31(d,6H)<br>2.0-2.3(q,2H)<br>6.28(d,1H)<br>6.7-7.4(m,7H)<br>7.7-7.9(t,1H) | 2950(2), 1745, 1595, 1580, 1490, 1445, 1200, 840, 820, 740 |
| XIV | 2(b) | 1.29(d,6H)<br>2.0-2.3(m,2H)<br>5.08(s,2H)<br>6.7(d,1H)<br>6.98(m,6H)<br>7.6-7.8(t,1H) | 3050, 2950, 1730, 1600, 1580, 1500, 835, 790, 705 |

Example 4

This Example illustrates the preparation of (RS)-1-cyano-1-(6-phenoxypyrid-2-yl)methyl (1R,*cis*)-3-(*Z*-2-chloro-3,3,3-trifluoroprop-1-en-1-yl)-2,2-dimethylcyclopropane carboxylate (Product XX) and its resolution into Products XVI and XVII.

Product XX (3.0 g) was obtained by reaction of (1R,*cis*)-3-(*Z*-2-chloro-3,3,3-trifluoroprop-1-en-1-yl)-2,2-dimethylcyclopropane carboxylic acid (1.88 g) and (RS)-1-cyano-1-(6-phenoxypyrid-2-yl)methanol (1.75 g) under the conditions of Example 2(b) above.

Separation of the two isomers was optimised using an analytical h.p.l.c. apparatus comprising a Partisil 5 column (dimensions 125 × 4.9 mm) with, as mobile phase, a *n*-hexane/diethyl ether mixture (84:16 by volume), and a flow rate of 1 $cm^3$ $min^{-1}$. The two isomers had retention times of 56.5 min and 68.5 min respectively. Separation of larger quantities was achieved using a Waters Preparative Liquid Chromatography System 500 employing a PrepPak cartridge, the same mobile phase as above, and a flow rate of 200 $cm^2$ $min^{-1}$. The whole sample (ca. 3.0 g) was injected and the main peaks recycled for a total of four passes, shaving the front of the first peak and the tail of the second peak each time. Two fractions were collected, a faster moving (0.96 g) and slower moving (1.02 g). The purity was checked by gas liquid chromatography. The faster moving fraction was shown to be a mixture of 3 parts of (S)-1-cyano-1-(6-phenoxypyrid-2-yl)methyl (1R,*cis*)-3-(*Z*-2-chloro-3,3,3-trifluoroprop-1-en-1-yl)-2,2-dimethylcyclopropane carboxylate and 47 parts of (R)-1-cyano-1-(6-phenoxypyridin-2-yl)methyl (1R,*cis*)-3-(*Z*-2-chloro-3,3,3-trifluoroprop-1-en-1-yl)-2,2-dimethylcyclopropane carboxylate (Product XVII), and the slower moving fraction was shown to be an 84:12 mixture of these isomers (Product XVI).

| PRODUCT | NMR ($CDCl_3$)<br>(main component) | INFRA RED ($cm^{-1}$)<br>(Liquid film) |
|---|---|---|
| XVI | 1.21(s,3H)<br>1.32(s,3H)<br>2.0-2.36(m,2H)<br>6.31(s,1H)<br>6.7-7.5(m,8H)<br>7.7-7.84(t,1H) | 3100, 3000,<br>1740, 1600,<br>1580, 1500,<br>1450, 700 |
| XVII | 1.31(d,6H)<br>2.0-2.32(m,2H)<br>6.26 (s,1H)<br>6.7-7.5(m,8H)<br>7.66-7.84(t,1H) | 3100, 2980,<br>2960, 1740,<br>1600, 1580,<br>1500, 1455,<br>700 |

Example 5

By the preparation of h.p.l.c. techniques described in the previous Example Product IV was separated into two fractions, identified as Products XIX, and XVII, by n.m.r. spectroscopy.

| PRODUCT | NMR ($CDCl_3$) (main component) |
|---|---|
| XVIII | 1.32(d,6H)<br>2.0-2.26(m,2H)<br>6.26(s,1H)<br>6.7-7.52(m,8H)<br>7.64-7.84(t,1H) |
| XIX | 1.21(s,3H)<br>1.32(s,3H)<br>1.99-2.34(m,2H)<br>6.28(s,1H)<br>6.7-7.5(m,8H)<br>7.64-7.84(t,1H) |

Example 6

This Example illustrates the insecticidal properties of Product of this invention.

The activity of the Product was determined using a variety of insect pests. The Product was used in the form of liquid preparations containing 500, 100, or 10 parts per million (ppm) by weight of the Product. The preparations were made by dissolving the Product in a mixture of solvents consisting of 4 parts by volume of acetone and 1 part by volume of diacetone alcohol. The solutions were then diluted with water containing 0.01% by weight of a wetting agent sold under the trade name "LISSAPOL" NX until the liquid preparations contained the required concentration of the Product. "Lissapol" is a Registered Trade Mark.

The test procedure adopted with regard to each pest was basically the same and comprised supporting a number of the pests on a medium which was usually a host plant or a foodstuff on which the pests feed, and treating either or both the pests and the medium with the preparations. The mortality of the pests was then assessed at periods usually varying from one to three days after the treatment. Details are given in Table II.

The results of the tests are given in Table III for each of the products at the rate in parts per million given in the second column as a grading of mortality designated as A, B, C or N wherein A indicates 100% mortality of at an application rate of 10 ppm, B indicates 100% mortality at 100 ppm, C indicates 100% mortality at 500 ppm and N represents less than 100% mortality at 500 ppm.

In Table III the pest organism used is designated by a letter code and the pest species, the support medium or food, and the type and duration of test is given in Table II.

TABLE II

| CODE LETTERS (Table III) | TEST SPECIES | SUPPORT MEDIUM/FOOD | TYPE OF TEST | DURATION (days) |
|---|---|---|---|---|
| TU | *Tetranychus urticae* (red spider mites – adult) | French bean leaf | Contact | 3 |
| AF MV | *Aphis fabae* *Myzus persicae* (aphids) | Broad bean leaf | Contact | 2 |
| PX | *Plutella xylostella* (Diamond back moth, larvae) | Cabbage leaf | Contact | 6 |
| CP | *Chilo partellus* (maize stem bore) | Oil seed rape leaf | Residual | 3 |
| HV | *Heliothis viriscens* (tobacco budworm) | Cotton leaf | Residual | 3 |
| DB | *Diabrotica balteata* (rootworm larvae) | Filter paper/ maize seed | Residual | 3 |
| BG | *Blattella germanica* (cockroach nymphs) | Plastic pot | Residual | 3 |
| MD | *Musca domestica* (houseflies – adults) | Cotton wool/ sugar | Contact | 1 |

* "Contact" test indicates that both pests and medium were treated and "residual" indicates that the medium was treated before infestation with the pests.

TABLE III

| PRODUCT | TU | AF | MV | MD | BG | HV | CP | PX | DB |
|---|---|---|---|---|---|---|---|---|---|
| I | A | A | - | B | B | A | - | - | - |
| II | B | A | - | A | B | B | - | A | A |
| III | C | A | - | B | A | A | - | A | A |
| IV | A | A | - | A | B | A | - | A | A |
| V | C | A | - | C | C | B | - | - | A |
| VI | A | A | - | B | B | B | - | - | A |
| VII | A | A | - | B | B | B | - | - | B |
| VIII | A | - | - | B | N | B | - | - | C |
| IX | N | A | - | B | C | - | A | - | A |
| X | A | - | A | A | B | B | A | - | A |
| XI | B | - | A | B | C | A | - | - | A |
| XII | B | - | B | B | B | B | A | - | A |
| XIII | B | - | A | B | B | A | A | - | A |
| XIV | N | - | A | B | B | B | A | - | A |
| XV | B | - | A | A | A | A | A | - | A |
| XVI | A | - | A | A | A | A | A | - | - |
| XVII | B | - | A | A | A | A | A | - | - |
| XVIII | A | - | A | A | B | A | A | - | - |
| XIX | A | - | A | A | B | A | A | - | - |
| Prior Art Compound* | C | A | - | B | C | B | A | - | B |

* 6–phenoxypyrid–2–ylmethyl *cis/trans* –3–(2,2–dichlorovinyl)–2,2–dimethylcyclopropane carboxylate

Example 7

This Example illustrates properties of Products of the invention against the larval stage of the rootworm *Diabrotica balteata.* The Product under test was dissolved in acetone and the solution successively diluted with acetone until the required concentrations were obtained. 1.0 ml of the solution thus obtained is applied to a filter paper (9 cm diameter) which is air dried to allow the solvent to evaporate and then placed in a petri dish. 1.0 ml of water is added and 10 early second instar larval *Diabrotica balteata* are placed on the filter paper together with a germinating maize seed. A lid is placed on the dish which is stored at 25°C and 60% relative humidity for 72 hours after which time the mortality of the larvae is assessed.

The results of this test were subjected to probit analysis to determine the concentration of active ingredient in parts per million (ppm) required to give 50% mortality of the larvae ($LC_{50}$). This is set out in Table IV below. In this test the known compound α-cyano-6-phenoxy-2-pyridylmethyl 3-(2,2-dichlorovinyl)-2,2-dimethylcyclopropane carboxylate was inactive at a concentration of 50 ppm.

| PRODUCT | $LC_{50}$ (ppm) |
|---|---|
| III | 0.15 |
| II | 0.35 |
| V | 0.05 |

Example 8

The toxicity (by direct contact spraying) of the invention compounds relative to the known compound permethrin (3-phenoxybenzyl *cis/trans* 3-(2,2-dichlorovinyl)-2,2-dimethylcyclopropane carboxylate was determined for adult houseflies (*Musca domestica*) and mosquitos (*Aedes aegypti*). The results are set out in the following table.

| PRODUCT | Relative efficacy with respect to permethrin | |
|---|---|---|
| | Houseflies | Mosquitos |
| I | 0.6 | 4.1 |
| IV | 15.6 | 14.6 |
| III | 4.8 | 13.4 |
| II | 2.5 | 17.7 |

Example 9

The knock-down effect of the Products was demonstrated by use of the Kearns and March test. Twenty 4—6 day old female houseflies (*Musca domestica*), or twenty 2—6 day old mosquitos (*Aedes aegypti*), were released into as Kearns and March chamber of capacity 0.055 $cm^3$. The Product under test was diluted with acetone to the appropriate concentration (ppm) and 0.4 $cm^3$ sprayed into the chamber via two nozzles. The test was replicated and the $KT_{50}$ and $KT_{90}$ values at various rates of active ingredients are determined by probit analysis from observations of the number of insects knocked down after various times. The results are given in the following tables.

A. *Musca domestica* (125ppm)

| PRODUCT | $KT_{50}$ | $KT_{90}$ | PRODUCT | $KT_{50}$ | $KT_{90}$ |
|---|---|---|---|---|---|
| I | 1.7 | 2.8 | IX | 3.0 | 4.5 |
| II | 2.3 | 3.1 | X | 3.8 | 6.1 |
| III | 2.4 | 4.7 | XI | 3.1 | 6.0 |
| IV | 1.9 | 4.2 | XII | 1.8 | 3.1 |
| V | 6.8 | >10 | XIII | 5.5 | 8.2 |
| VI | 3.2 | 5.3 | XIV | 3.7 | 6.3 |
| VII | 3.9 | 7.1 | XV | 2.8 | 4.5 |
| VIII | 7.1 | >10 | | | |

B. *Aedes aegypti* (50ppm)

| Product | $KT_{50}$ | $KT_{90}$ |
|---|---|---|
| I | 2.1 | 3.5 |
| II | 3.3 | 7.0 |
| III | 3.4 | 6.1 |
| IV | 3.7 | 5.9 |
| V | 2.7 | 6.9 |

C. *Musca domestica* (1000, 500, 100, 25ppm)

| PRODUCT | I | | II | | IV | |
|---|---|---|---|---|---|---|
| Rate(ppm) | $KT_{50}$ | $KT_{90}$ | $KT_{50}$ | $KT_{90}$ | $KT_{50}$ | $KT_{90}$ |
| 1000 | 1.4 | 2.4 | 1.7 | 2.6 | 1.7 | 2.7 |
| 500 | 1.5 | 2.5 | 2.0 | 3.0 | 1.5 | 2.7 |
| 100 | 2.5 | 4.6 | 2.4 | 3.8 | 2.6 | 3.8 |
| 25 | 4.6 | >10 | 3.3 | 6.0 | 2.6 | 4.8 |

D. *Aedes Aegypt* (100, 25, 10, 5ppm)

| PRODUCT | I | | II | | IV | |
|---|---|---|---|---|---|---|
| Rate(ppm) | $KT_{50}$ | $KT_{90}$ | $KT_{50}$ | $KT_{90}$ | $KT_{50}$ | $KT_{90}$ |
| 100 | 1.5 | 3.0 | 6.4 | >10 | 1.8 | 4.3 |
| 25 | 2.4 | 3.8 | >10 | >10 | 3.9 | 6.8 |
| 10 | 2.7 | 4.0 | >10 | >10 | 5.8 | 9.0 |
| 5 | 3.3 | 6.4 | >10 | >10 | 5.2 | 9.0 |

Example 10

The knock-down effect was also demonstrated against cockroaches by the following test.

Ten adult male *Blatella germanica* were placed in an open ended plastic container of dimensions 10 × 7 cm and contained therein by fabric mesh netting (2 mm mesh). The Product under test was diluted with water containing 0.1% by weight of a wetting agent sold under the name of 'Synperonic' NX (which is a condensate of nonyl phenol with ethylene oxide) until the desired concentration of Product was obtained. The preparation (2.0 $cm^3$) was then sprayed over the container and the $KT_{50}$ and $KT_{90}$ values calculated from observations of the number of cockroaches knocked down at various times. The test was replicated and the results are given in the following tables.

A. *Blatella germanica* (1000ppm)

| Product | $KT_{50}$ | $KT_{90}$ |
|---|---|---|
| I | 7.4 | 13.5 |
| II | 14 | 28 |
| III | 8.3 | 20 |
| IV | 5.3 | 11.2 |
| Tetramethrin | $>$30 | $>$30 |

Tetramethrin is a recommended knock-down agent for incorporation in insecticidal formulations.

B. *Blatella germanica* (500ppm)

| Product | $KT_{50}$ | $KT_{90}$ |
|---|---|---|
| I | 19 | 38 |
| II | 14 | 28 |
| III | 7.7 | 14.5 |
| IV | 7.2 | 20 |
| Prior art Compound ** | 9.6 | 26 |

** (RS)–1–cyano–1–(6–phenoxypyrid–2–yl)methyl (1RS,*cis*)/(1RS, *trans*)–3–(2,2–dichlorovinyl)–2,2–dimethylcyclo–propane carboxylate.

**Claims**

1. Compounds of formula:

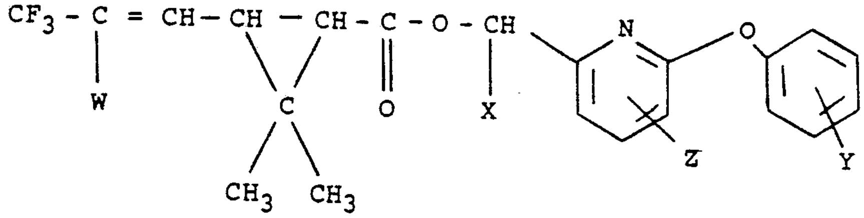

wherein W is chloro, bromo or fluoro, X is hydrogen, cyano or ethynyl, Y is hydrogen, chloro, fluoro or bromo and Z is hydrogen, chloro or fluoro.

2. Compounds according to claim 1 wherein W is chloro, bromo or fluoro, X is hydrogen or cyano, Y is hydrogen, fluoro or chloro and Z is hydrogen.

3. Compounds according to claim 2 wherein Y is hydrogen.

4. Compounds according to claim 3 wherein W is chloro.

5. Compounds according to claim 1 selected from the group of compounds consisting of

6-phenoxypyrid-2-ylmethyl (1RS,*cis*)-3-(*Z*-2-chloro-3,3,3-trifluoroprop-1-en-1-yl)-2,2-dimethylcyclopropane carboxylate,

(RS)-1-cyano-1-(6-phenoxypyrid-2-yl)methyl (1RS,*cis*)-3-(*Z*-2-chloro-3,3,3-trifluoroprop-1-en-1-yl)-2,2-dimethylcyclopropane carboxylate,

6-(4-fluorophenoxy)pyrid-2-ylmethyl (1RS,*cis*)-3-(*Z*-2-chloro-3,3,3-trifluoroprop-1-en-1-yl)-2,2-dimethylcyclopropane carboxylate,

6-phenoxypyrid-2-ylmethyl (1RS,*cis*)-3-(*Z*-2-bromo-3,3,3-trifluoroprop-1-en-1-yl)-2,2-dimethylcyclopropane carboxylate, and

(RS)-1-cyano-1-(6-phenoxypyrid-2-yl)methyl (1RS,*cis*)-3-(*Z*-2,3,3,3-trifluoroprop-1-en-1-yl)-2,2-dimethylcyclopropane carboxylate.

6. An insecticidal composition comprising an insecticidally effective amount of a compound according to claim 1.

7. An insecticidal composition for combating flying insect pests comprising a knock down effective amount of a compound according to claim 1.

8. A method of combating insect pests at a locus which comprises treating the locus with an insecticidally effective amount of a composition according to claim 6.

9. A process for preparing a compound according to claim 1 which comprises reacting a compound of formula:

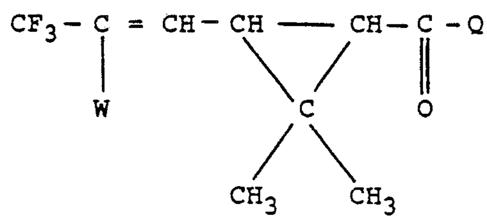

with a compound of formula:

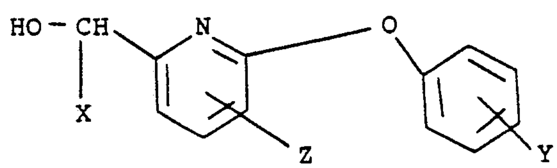

wherein W, X, Y and Z have any of the meanings given in claim 1 and wherein either (i) Q represents the hydroxy group and the reaction is carried out in the presence of a dehydrating agent or (ii) Q is halo and the reaction is carried out in the presence of a base.

**Patentansprüche**

1. Verbindungen der Formel

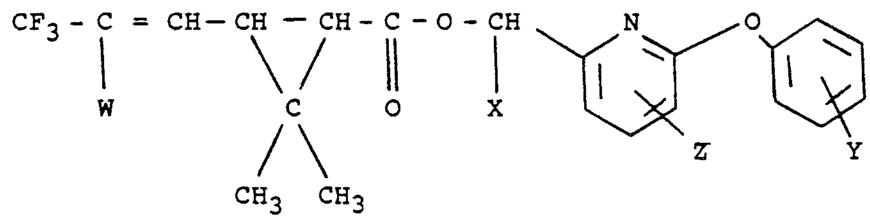

,

worin W für Chloro, Bromo oder Fluoro steht, X für Wasserstoff, Cyano oder Ethinyl steht, Y für Wasserstoff, Chloro, Fluoro oder Bromo steht und Z für Wasserstoff, Chloro oder Fluoro steht.

2. Verbindungen nach Anspruch 1, bei welchen Y für Chloro, Bromo oder Fluoro steht, X für Wasserstoff oder Cyano steht, Y für Wasserstoff, Fluoro oder Chloro steht und Z für Wasserstoff steht.

3. Verbindungen nach Anspruch 2, bei welchen Y für Wasserstoff steht.

4. Verbindungen nach Anspruch 3, bei welchen W für Chloro steht.

5. Verbindung nach Anspruch 1, welche ausgewählt ist aus der folgenden Gruppe von Verbindungen:

(1RS,cis)-3-(Z-2-Chloro-3,3,3-trifluoroprop-1-en-1-yl)-2,2-dimethylcyclopropan-carbonsäure-6-phenoxypyrid-2-ylmethyl-ester,

(1RS,cis)-3-(Z-2-Chloro-3,3,3-trifluoroprop-1-en-1-yl)-2,2-dimethylcyclopropan-carbonsäure-(RS)-1-cyano-1-(6-phenoxypyrid-2-yl)methyl-ester,

(1RS,cis)-3-(Z-2-Chloro-3,3,3-trifluoroprop-1-en-1-yl)-2,2-dimethylcyclopropan-carbonsäure-6-(4-fluorophenoxy)pyrid-2-ylmethyl-ester,

(1RS,cis)-3-(Z-2-Bromo-3,3,3-trifluoroprop-1-en-1-yl)-2,2-dimethylcyclopropan-carbonsäure-6-phenoxypyrid-2-ylmethyl-ester und

(1RS,cis)-3-(Z-2,3,3,3-Tetrafluoroprop-1-en-1-yl)-2,2-dimethylcyclopropan-carbonsäure-(RS)-1-cyano-1-(6-phenoxypyrid-2-yl)methyl-ester.

6. Insecticide Zusammensetzung, welche eine insecticid wirksame Menge einer Verbindung nach Anspruch 1 enthält.

7. Insecticide Zusammensetzung zur Bekämpfung von fliegenden Insektenschädlingen, welche eine zur Erzielung eines "knockdown-Effekts" wirksame Menge einer Verbindung nach Anspruch 1 enthält.

8. Verfahren zur Bekämpfung von Insektenschädlingen an einem Ort, bei welchem der Ort mit einer insecticid wirksamen Menge einer Zusammensetzung nach Anspruch 6 behandelt wird.

9. Verfahren zur Herstellung einer Verbindung nach Anspruch 1, bei welcher eine Verbindung der Formel

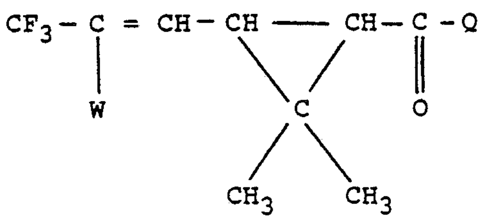

mit einer Verbindung der Formel

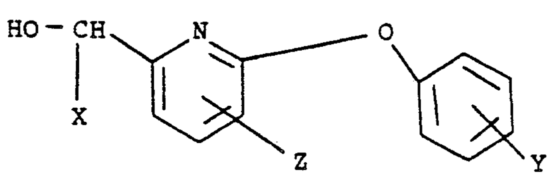

umgesetzt wird, wobei W, X, Y und Z die in Anspruch 1 angegebenen Bedeutungen besitzen und wobei entweder (i) Q für die Hydroxygruppe steht und die Reaktion in Gegenwart eines Entwässerungsmittels ausgeführt wird oder (ii) Q für Halogen steht und die Reaktion in Gegenwart einer Base ausgeführt wird.

**Revendications**

1. Composés répondant à la formule:

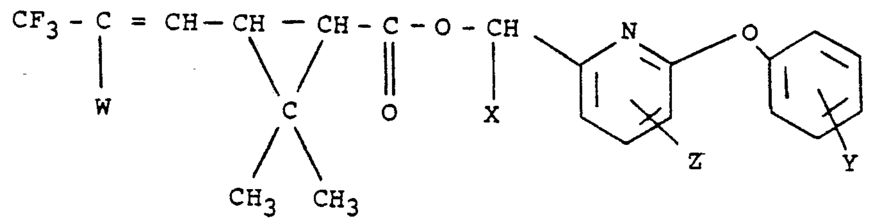

dans laquelle W représente le chlore, le brome ou le fluor, X représente l'hydrogène, un groupe cyano ou éthynyle, Y représente l'hydrogène, un groupe chloro, fluoro ou bromo et Z représente l'hydrogène, un groupe chloro ou fluoro.

2. Composés suivant la revendication 1, dans lesquels W représente le chlore, le brome ou le fluor, X représente l'hydrogène ou un groupe cyano, Y représente l'hydrogène, le fluor ou le chlore et Z représente l'hydrogène.

3. Composés suivant la revendication 2, dans lesquels Y représente l'hydrogène.

4. Composés suivant la revendication 3, dans lesquels W représente le chlore.

5. Composés suivant la revendication 1, choisis dans le groupe de composés comprenant

le (1RS,*cis*)-3-(*Z*-2-chloro-3,3,3-trifluoroprop-1-ène-1-yl)-2,2-diméthylcyclopropane-carboxylate de 6-phénoxypyrid-2-ylméthyle,

le (1RS,*cis*)-3-(*Z*-2-chloro-3,3,3-trifluoroprop-1-ène-1-yl)-2,2-diméthylcyclopropane-carboxylate de (RS)-1-cyano-1-(6-phénoxypyrid-2-yl)-méthyle,

le (1RS,*cis*)-3-(*Z*-2-chloro-3,3,3-trifluoroprop-1-ène-1-yl)-2,2-diméthylcyclopropane-carboxylate de 6-(4-fluorophénoxy)pyrid-2-ylméthyle,

le (1RS,*cis*)-3-(*Z*-2,3,3,3-trifluoroprop-1-ène-1-yl)-2,2-diméthylcyclopropane-carboxylate de 6-phénoxypyrid-2-ylméthyle, et

le (1RS,*cis*)-3-(*Z*-2-bromo-3,3,3-tétrafluoroprop-1-ène-1yl)-2,2-diméthylcyclopropane-carboxylate de 6-phénoxypyrid-2-ylméthyle, et

le (1RS,*cis*)-3-(*Z*-2,3,3,3-tétrafluoroprop-1-ène-1-yl)-2,2-diméthylcyclopropane-carboxylate de (RS)-1-cyano-1-(6-phénoxypyrid-2-yl)-méthyle.

6. Composition insecticide comprenant une quantité à effet insecticide d'un composé suivant la revendication 1.

7. Composition insecticide destinée à lutter contre des insectes volants parasites, comprenant une quantité à effet de choc d'un composé suivant la revendication 1.

8. Procédé pour lutter contre des insectes parasites présents dans un milieu, qui consiste à traiter le milieu avec une quantité à effet insecticide d'une composition suivant la revendication 6.

9. Procédé de préparation d'un composé suivant la revendication 1, qui consiste à faire réagir un composé répondant à la formule:

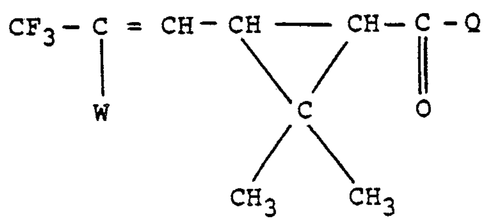

avec un composé répondant à la formule:

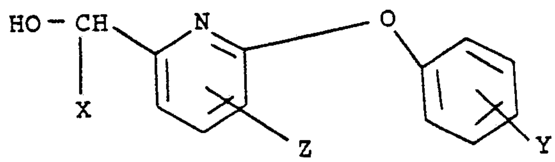

formules dans lesquelles W, X, Y et Z répondent à l'une quelconque des définitions mentionnées dans la revendication 1, et (i) Q représente un groupe hydroxy et la réaction est conduite en présence d'un agent déshydratant, ou bien (ii) Q représente un atome d'halogène et la réaction est conduite en présence d'une base.